(19) Europäisches Patentamt
European Patent Office
Office européen des brevets

(11) Publication number: **0 045 088**
**B1**

(12) **EUROPEAN PATENT SPECIFICATION**

(45) Date of publication of patent specification: **20.06.84**

(21) Application number: **81105930.2**

(22) Date of filing: **27.07.81**

(51) Int. Cl.³: **C 07 C 121/66,**
C 07 C 120/00,
C 07 C 87/30

(54) **Process for preparing substituted p-chlorophenyl aliphatic nitriles.**

(30) Priority: **28.07.80 US 172790**

(43) Date of publication of application:
**03.02.82 Bulletin 82/5**

(45) Publication of the grant of the patent:
**20.06.84 Bulletin 84/25**

(84) Designated Contracting States:
**AT BE CH DE FR GB IT LI NL**

(56) References cited:
**DE - A - 2 737 945**

**Chemical Abstracts vol. 84, no. 25, 1976 Columbus, Ohio, USA G.W. FISCHER et al. "Reactivity and toxicity of cyclic sulfuric acid esters" page 130, abstract no. 174678b**

(73) Proprietor: **STAUFFER CHEMICAL COMPANY**
**Westport Connecticut 06880 (US)**

(72) Inventor: **Felix, Raymond Anthony**
**1662 Shasta Street**
**Richmond California 94804 (US)**

(74) Representative: **Jaeger, Klaus, Dr.rer.nat. Dipl.-Chem. et al,**
**Jaeger, Grams & Pontani Patentanwälte**
**Bergstrasse 48 1/2**
**D-8035 München-Gauting (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 045 088 B1

**Description**

Background of the Invention

3-Methyl-2-p-chlorophenyl butyronitrile is useful as an intermediate for preparing the (±)-cyano(3-phenoxyphenyl)methyl ester of (±)-3-methyl-2-(4-chlorophenyl)butanoic acid which is useful as an insecticide and acaricide composition and has the chemical structure

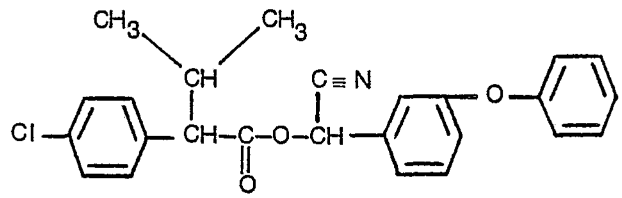

This compound can be prepared by reacting 3-methyl-2-p-chlorophenyl butyric acid chloride with m-phenoxybenzaldehyde cyanohydrin in the presence of an acid scavenger such as triethylamine. 3-Methyl-2-p-chlorophenyl butyric acid chloride can be prepared from 3-methyl-2-p-chlorophenyl butyronitrile by hydrolyzing the nitrile to an acid and converting the acid to an acid chloride by standard known procedures.

A method for preparing 3-methyl-2-(4-halogen phenyl)-butyronitrile by reacting p-halogenphenyl-acetonitrile with an isopropylhalide in the presence of a quarternery organoammonium salt as catalyst is described in the DE—A—2 737 945. A prerequisite of this method is the purity of the starting materials.

Detailed Description of the Invention

3-Alkyl-2-p-chlorophenyl butyronitrile can be prepared by reacting p-chlorophenyl acetonitrile with a lower alkyl sulfonate of a lower alkyl alcohol under phase transfer catalytic conditions in the presence of a 40 weight percent to saturated basic solution such as 50% NaOH or 50% KOH. Any number of phase transfer catalysts can be used such as tetrabutylphosphonium chloride, benzyltriethylammonium chloride, tricaprylmethylammonium chloride, tetrabutylammonium chloride, or cryptates or crown ethers such as 18-crown-6. The amount of phase transfer catalyst present can range from 1/2 to 10 weight percent. The reaction can be conducted at a temperature from 0°C to 200°C, preferably from 50°C to 100°C. Reaction times are from one-half hour to 48 hours, preferably two to three hours depending on reaction temperature. The reaction rate depends upon reaction temperature. The reaction is as follows:

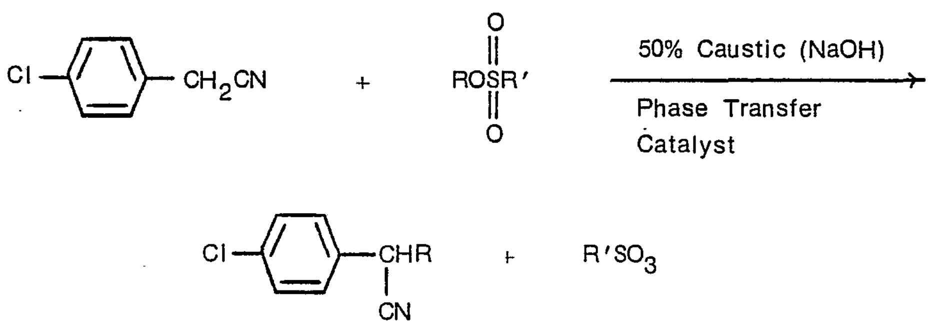


wherein R can be $C_1$—$C_5$ primary or secondary alkyl such as methyl, ethyl, propyl, isopropyl, butyl, isobutyl, pentyl and isopentyl and R′ can be primary, secondary or tertiary $C_1$—$C_5$ such as methyl, ethyl, propyl, isopropyl, butyl, isobutyl, t-butyl, pentyl isopentyl or t-pentyl. The above process has an additional advantage in that the alkyl sulfonate of the alkyl alcohol are high boiling (~200°C) and do not require pressure equipment during the reaction as do lower alkyl chlorides (RCl wherein R is as defined above) whose boiling point is about 35°C.

The process of this invention is exemplified by the following example.

Example I

3-methyl-2-p-chlorophenyl butyronitrile

In a one liter flask was placed 135 grams (g) (0.9 mole (m)) of p-chlorophenyl acetonitrile and 138 g (1 m) of isopropyl methanesulfonate. To this was added in one portion 300 milliliters (ml) of 50% NaOH and 30 ml of 40% tetrabutylammonium hydroxide. The mixture was heated on a stream bath

with vigorous stirring for three hours. Two liters of water was then added and the mixture extracted three times with 200 ml portions of $CH_2Cl_2$. The organic layers were combined, dried with $MgSO_4$ and evaporated. A quantitative yield of 3-methyl-2-p-chlorophenyl butyronitrile was obtained. The product was identified by infrared and nuclear magnetic resonance spectra as the title compound having the structural formula

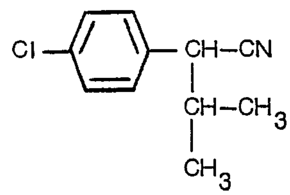

## Claims

1. A process for preparing a substituted p-chlorophenyl aliphatic nitrile having the formula

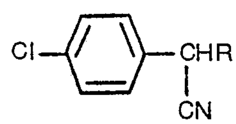

wherein R is a $C_1$—$C_5$ primary or secondary lower alkyl selected from the group consisting of methyl, ethyl, propyl, isopropyl, butyl, isobutyl, pentyl and isopentyl comprising reacting chlorophenyl acetonitrile with a lower alkyl sulfonate of a lower alkyl alcohol having the formula

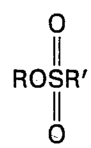

wherein R is a $C_1$—$C_5$ primary or secondary lower alkyl selected from the group consisting of methyl, ethyl, propyl, isopropyl, butyl, isobutyl, pentyl and isopentyl and R' is a $C_1$—$C_5$ lower alkyl selected from the group consisting of methyl, ethyl, propyl, isopropyl, butyl, isobutyl, t-butyl, pentyl, isopentyl or t-pentyl in the presence of a base and a phase transfer catalyst.

2. The process of Claim 1 wherein R is isopropyl.

3. The process of Claims 1 or 2 wherein the base is selected from the group consisting of 50% NaOH and 50% KOH, the reaction temperature is from 0°C to 200°C and the phase transfer catalyst is selected from the group consisting of tetrabutylphosphonium chloride, benzyltriethylammonium chloride, tricaprylmethylammonium chloride or tetrabutylammonium chloride.

## Revendications

1. Un procédé de préparation d'un (p-chlorophényl)nitrile aliphatique substitué ayant la formule:

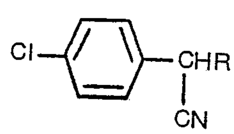

dans laquelle:

R est un radical alkyle inférieur primaire ou secondaire, comprenant 1 à 5 atomes de carbone, choisi dans le groupe comprenant les radicaux méthyle, éthyle, propyle, isopropyle, butyle, isobutyle, pentyle et isopentyle, consistant à faire réagir le p-chlorophényl acétonitrile avec un alkyl(inf) sulfonate d'un alcool alkylique inférieur ayant la formule:

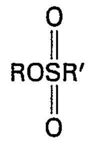

dans laquelle:

R est un radical inférieur, primaire ou secondaire, choisi dans le groupe comprenant les radicaux méthyle, éthyle, propyle, isopropyle, butyle, isobutyle, pentyle et isopentyle; et,

R' est un radical alkyle inférieur comprenant 1 à 5 atomes de carbone, choisi dans le groupe comprenant les radicaux méthyle, éthyle, propyle, isopropyle, butyle, isobutyle, t-butyle, pentyle, isopentyle ou t-pentyle, en présence d'un base et d'un catalyseur de transfert de phase.

2. Le procédé selon la revendication 1, dans lequel R est un radical isopropyle.

3. Le procédé selon la revendication 1 ou 2, dans lequel la base est choisie dans le groupe comprenant NaOH à 50% et KOH à 50%, la température de la réaction étant comprise entre 0°C et 200°C et le catalyseur de transfert de phase est choisi dans le groupe comprenant le chlorure de tétrabutylphosphonium, le chlorure de benzyltriéthylammonium, le chlorure de tricaprylméthylammonium et le chlorure de tétrabutylammonium.

**Patentansprüche**

1. Verfahren zur Herstellung eines substituierten p-Chlorphenylderivates von aliphatischen Nitrilen der Formel

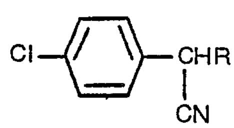

worin R ein primärer oder sekundärer $C_1$—$C_5$-Niederalkylrest aus der Gruppe von Methyl, Ethyl, Propyl, Isopropyl, Butyl, Isobutyl, Pentyl und Isopentyl ist, wobei ein p-Chlorphenylacetonitril mit einem niederen Alkylsulfonat eines niederen Alkylalkohols der Formel

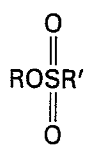

worin R ein primärer oder sekundärer $C_1$—$C_5$-Niederalkylrest aus der Gruppe von Methyl, Ethyl, Propyl, Isopropyl, Butyl, Isobutyl, Pentyl und Isopentyl ist, und R' ein $C_1$—$C_5$-Niederalkylrest aus der Gruppe von Methyl, Ethyl, Propyl, Isopropyl, Butyl, Isobutyl, tert.-Butyl, Pentyl, Isopentyl und tert-Pentyl ist, in Gegenwart einer Base und eines Phasentransfer-Katalysators umgesetzt wird.

2. Verfahren nach Anspruch 1, worin R Isopropyl ist.

3. Verfahren nach Anspruch 1 oder Anspruch 2, worin die Base aus der Gruppe von 50% NaOH und 50% KOH ausgewählt ist, die Reaktionstemperatur im Bereich von 0°C bis 200°C liegt und der Phasentransfer-Katalysator aus der Gruppe von Tetrabutylphosphoniumchlorid, Benzyltriethylammoniumchlorid, Tricaprylmethylammoniumchlorid und Tetrabutylammoniumchlorid ausgewählt ist.